# FASCICULE DE BREVET EUROPEEN

(11) **EP 1 224 182 B1**
(45) Date de publication et mention de la délivrance du brevet: **16.07.2003**
(21) Numéro de dépôt: 00969659.2
(22) Date de dépôt: 20.10.2000
(51) Int. Cl.: C07D 405/04, C07D 409/04, C07D 401/04, A61K 31/4725, A61P 25/00

(54) **1-(BUTYROLACTONE)-ISOQUINOLINES N-SUBSTITUEES POUR LE TRAITEMENT DES TROUBLES NERVEUX**
N-SUBSTITUIERTE 1-(BUTYROLACTON)-ISOCHINOLINE ZUR BEHANDLUNG VON NERVENSTÖRUNGEN
N-SUBSTITUTED 1-(BUTYROLACTONE)-ISOQUINOLINES FOR TREATING NERVOUS DISORDERS

(30) Priorité: 22.10.1999 FR 9913232
(43) Date de publication de la demande: 24.07.2002
(73) Titulaire: CENTRE NATIONAL DE LA RECHERCHE SCIENTIFIQUE (CNRS), 75794 Paris Cedex 16 (FR); Innovationsagentur GmbH bu Technology Marketing Austria (TECMA), 1020 Vienne (AT); UNIVERSITE DE BERNE, CH-3010 Berne (CH)
(72) Inventeur: DODD, Robert, F-75009 Paris (FR); RAZET, Rodolphe, F-94220 Charenton-le-Pont (FR); POTIER, Pierre, Jean-Paul, F-75007 Paris (FR); SIEGHART, Werner, A-1190 Vienne (AT); JURSKY, Frantisek, Bratislava (SK); FURTMULLER, Roman, A-3143 Pyhra (AT); SIGEL, Erwin, CH-3047 Bremgarten (CH); THOMET, Urs, CH-4114 Hofstetten (CH)
(74) Mandataire: Warcoin, Jacques
(86) Numéro de dépôt international: FR0002927
(87) Numéro de publication internationale: WO01029030

(56) Documents cités:
- EP-A- 0 419 247
- FR-A- 2 750 427
- P. KROGSGAARD-LARSEN ET AL: "Gaba(A) receptor agonists, partial agonists and antagonists. Design and therapeutic prospects" JOURNAL OF MEDICINAL CHEMISTRY, vol. 37, no. 16, 1994, pages 2489-2505, XP002141451 WASHINGTON US cité dans la demande
- CHEMICAL ABSTRACTS, vol. 117, no. 21, 23 novembre 1992 (1992-11-23) Columbus, Ohio, US; abstract no. 205616, MINCHIN M.C.W. ET AL: "A novel GABA-like autoreceptor modulates GABA release" XP002159129 & MOL. NEUROPHARMACOL., vol. 2, no. 2, 1992, pages 137-139,

## Description

La présente invention concerne des 1-(2-butyrolactones et 2-thiobutyrolactones)-isoquinolines N-substituées, leur préparation, les compositions pharmaceutiques les comprenant et leur utilisation comme stimulant de l'activité de l'acide γ-aminobutyrique et comme médicament destiné de préférence au traitement des troubles nerveux.

L'acide γ-aminobutyrique (ou GABA (1)) est le neurotransmetteur inhibiteur le plus important du système nerveux central. Il agit au niveau de trois classes distinctes de récepteurs dénommés récepteurs GABA-A, GABA-B et GABA-C. Le récepteur GABA-A, dont la séquence d'acides aminés a été déterminée par des techniques de clonage, est une structure pentamérique composée de sous-unités α, β, γ, δ et/ou ρ. Jusqu'à présent, 6 sous-unités α, 3 sous-unités β, 3 sous-unités γ, 1 sous-unité δ et 2 sous-unités ρ ont été identifiées et séquencées. Cinq de ces sous-unités (par exemple 2α₁ 2β₂ γ₂) se rassemblent pour former un canal perméable aux ions chlorure. En se liant à ce récepteur GABA-A, le GABA accroît la perméabilité du canal aux ions chlorure, inhibant ainsi la transmission neuronale. Au vu du grand nombre de permutations possibles des diverses sous-unités, une très grande hétérogénéité du récepteur GABA-A est observée dans le cerveau des mammifères et différentes structures du cerveau montrent généralement une prépondérance pour certaines combinaisons de sous-unités.

La recherche de ligands sélectifs de l'une de ces différentes sous-classes de récepteurs GABA-A est un objectif majeur de la recherche clinique médicale dans ce domaine.

En dehors du GABA, on connaît un grand nombre de différentes classes de composés se liant au récepteur GABA-A. Certains produits, tels que le muscimol et l'isoguvacine, se lient directement au même site que le GABA sur le récepteur GABA-A et stimulent le récepteur de la même façon que le GABA lui-même. A l'opposé de ces agonistes, certaines substances, comme la bicuculline (**2**), inhibent de façon compétitive l'action du GABA. De tels antagonistes du récepteur du GABA montrent des propriétés convulsivantes *in vivo* (P. Krogsgaard-Larsen, B. Frolund, F.S. Jorgensen, A. Schousboe, J. Med. Chem., 1994, 37, 2489).

L'action inhibitrice du GABA peut être modulée par des composés qui interagissent avec une variété de sites allostériques sur le récepteur GABA-A distincts du site de reconnaissance du GABA. Une des classes les plus connues de modulateurs allostériques du récepteur GABA-A est celle des benzodiazépines (par exemple le diazépam (**3**)). En se liant ainsi à leur propre site de reconnaissance sur le récepteur GABA-A (le récepteur des benzodiazépines ou BZR), ces composés améliorent l'action du GABA en augmentant la fréquence d'ouverture du canal chlorure (R.E. Study, J.L. Barker, Proc. Natl. Acad. Sci. USA, 1981, 78, 7180). Il en résulte les activités anticonvulsivante, anxiolytique, sédative-hypnotique et myorelaxante de ces produits largement utilisés en clinique. D'autres classes de composés structurellement non apparentées aux benzodiazépines, telles que les triazolopyridazines (par exemple Cl 218872 (**4**)), les imidazopyridines (par exemple le zolpidem (**5**)), les cyclopyrrolones (par exemple le zopicolone (**6**)) et les β-carbolines (par exemple le β-CCM (**7**)), peuvent également se lier aux récepteurs des benzodiazépines. Dans le cas de ces derniers, certains dérivés inhibent, plutôt qu'augmentent, l'action neuro-inhibitrice du GABA (R.L. Macdonald, R.E. Twyman in «Ion Channels» ed. by T. Narahashi, Vol. 3, pp. 315-343, Plenum Press, New York, 1992). Dans ce cas, les composés, généralement convulsivant, sont appelés agonistes inverses (ou modulateurs allostériques négatifs) du BZR, pour les distinguer des agonistes (ou modulateurs allostériques positifs) du BZR utiles thérapeutiquement. Certains de ces produits font preuve d'une sélectivité au niveau des diverses sous-classes de récepteurs GABA-A/benzodiazépines. Ainsi le zolpidem, utilisé cliniquement comme hypnotique, est sélectif pour la sous-classe de récepteurs des benzodiazépines que l'on trouve de façon prépondérante dans le cervelet (récepteurs BZ1) (S. Arbilla, H. Depoortere, P. George, S.Z. Langer, Naunyn-Schmiedeberg's Arch. Pharmacol., 1985, 330, 248). Cette sélectivité se traduit soit par un spectre d'activité plus étroit (par exemple, anxiolyse sans effet hypnotique) soit par une diminution des effets indésirables de ce type de produit (accoutumance, dépendance, amnésie ...).

D'autres sites existent sur le récepteur GABA-A qui permettent également, en fonction de sa liaison avec une molécule appropriée, de moduler l'activité du GABA. Parmi ces sites, citons ceux pour les neurostéroïdes (par exemple la 3α-OH-5α-pregnane-20-one), les barbituriques (par exemple le pentobarbital), les anesthésiants (par exemple le propofol), les convulsivants de cage t-butylbicyclophosphorothionate qui se lient au site de la picrotoxine du récepteur GABA-A (W. Sieghart, Pharmacol. Rev., 1995, 47, 181 et C.R. Gardner, W.R. Tully, C.J.R. Hedgecock, Prog. Neurobiol., 1993, 40, 1). D'autres sites de liaison, moins bien caractérisés mais apparemment distincts, sont ceux du loreclézole et des γ-butyrolactones. De tels composés modulent aussi positivement l'action du GABA et cet effet est traduit en une action in vivo anticonvulsivante et/ou anxiolytique.

Il est ainsi clair qu'un grand nombre de sites modulatoires allostériques, qui peuvent augmenter l'action du GABA et ainsi démontrer une efficacité thérapeutique dans une large gamme de désordres du système nerveux central, existent sur le récepteur GABA-A. Il peut ainsi être raisonnablement conclu que de nouvelles structures chimiques peuvent découvrir d'autres sites modulatoires allostériques à ce jour non caractérisés sur le récepteur GABA-A ou se lier à des sites connus avec de plus grandes affinités ou de plus grandes sélectivités. De tels composés peuvent, comme résultat, démontrer une activité puissante et/ou hautement spécifique de même que de plus faibles effets secondaires indésirables dans le traitement de tels désordres.

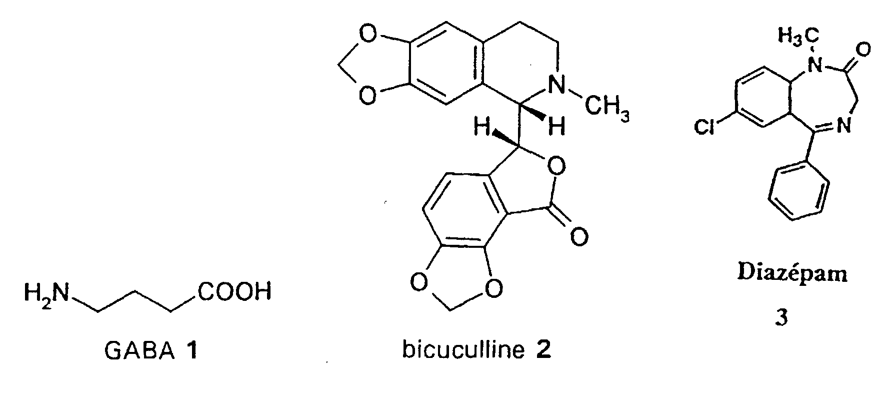

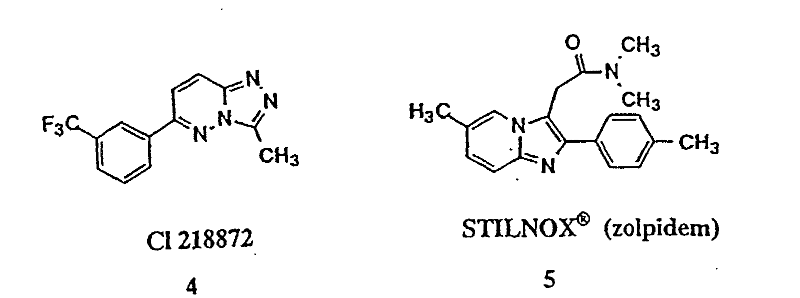

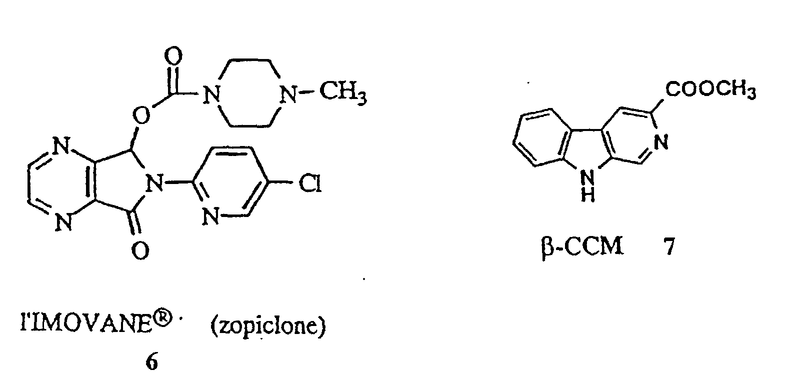

Les laboratoires Wyeth revendiquent que le composé **17** obtenu selon le schéma 1 (et les composés apparentés) sont des antagonistes de l'auto-récepteur GABA et sont ainsi utiles pour le traitement des désordres du système nerveux central et de la douleur (Brevet européen n° EP0419247A2). L'autorécepteur GABA est considéré comme étant une entité pharmacologique distincte du récepteur GABA-A lui-même. Les composés de type **17** n'ont ainsi aucune action ou une très faible action sur des récepteurs GABA-A. Les ligands de l'autorécepteur GABA montrent des exigences structurelles différentes de celles du récepteur GABA-A.

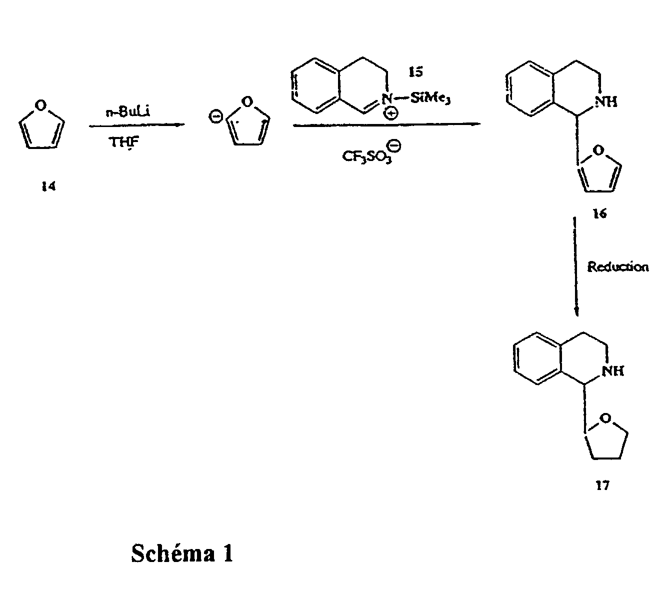

La Bicuculline **2**, dont la structure moléculaire a été utilisée comme point de départ pour la conception des composés de la présente invention, est un produit naturel, isolé à partir de la Dicentra cucullaria en 1932 par Manske (R.H. Manske, Can. J. Res., 1932, 7, 265). Ce composé, un convulsivant puissant, est un antagoniste compétitif du récepteur GABA-A, se liant au même site que le GABA lui-même (M.A. Simmonds, Br. J. Pharmacol., 1978, 63, 495). A cause de la nature convulsivante de la bicuculline, très peu d'études de structure-fonction ont été conduites sur cette molécule. Allan et Apostopoulos (R.D. Allan, C. Apostopoulos, Aust. J. Chem., 1990, 43, 1259) ont décrit la préparation des dérivés de la bicuculline modifiés à la position C-9 en partant de la 9-hydroxyméthyl bicuculline dont la synthèse a été décrite par Bhattacharyya et al (A. Bhattacharyya, K.M. Madyastha, P.K. Bhattacharyya, M.S. Devanandan, Indian J. Biochem. Biophys., 1981, 18, 171). Aucune donnée biologique n'a été reportée. Simonyi et al. (J. Kardos, G. Blasko, P. Kerekes, I. Kovacs, M. Simonyi, Biochem. Pharmacol., 1984, 33, 3537) ont déterminé les activités du récepteur GABA-A de 45 alcaloïdes phthalidéisoquinolines apparentés à la (+)-bicuculline. Aucun composé n'était plus actif que la bicuculline mais dans tous les cas le stéréoisomère *érythro* (c'est à dire celui de la bicuculline) était plus actif que l'isomère *thréo*. Cette conclusion a été confirmée par les mêmes auteurs dans des études apparentées publiées en 1996 (J. Kardos, T. Blandl, N.D. Luyen, G. Dornyei, E. Gacs-Baitz, M. Simonyi, D.J. Cash, G. Blasko, C. Szantay, Eur. J. Med. Chem., 1996, 31, 761). L'importance du cycle fusionné « A » de la bicuculline pour l'activité du récepteur GABA-A n'a jamais été démontrée, sauf dans la présente invention. Alors que la bicuculline était le point de départ de notre étude, les molécules les plus actives synthétisées diffèrent considérablement de la bicuculline en ce que :
- le cycle fusionné « A » est absent et les liaisons 3,4 sont saturées,
- les isomères *thréo* plutôt *qu'érythro* sont les plus actifs,
- le cycle aromatique benzo de l'entité isoquinoline n'est pas substitué,
- l'atome d'azote de l'entité isoquinoline est substitué par un carbamate d'aryle un sulfonate d'aryle plutôt qu'un groupe méthyle.

Finalement, les composés de la présente invention se lient au mieux seulement faiblement au site du GABA sur le récepteur GABA-A (ainsi mesuré par les études de déplacement du H³-muscimol) mais plutôt, se lient au site des benzodiazépines du récepteur GABA-A ou sur un site encore non identifié de ce récepteur ou, dans certains cas, sur ces deux derniers sites. De façon plus importante, alors que la bicuculline et ces analogues connus inhibent les courants produits par le GABA dans les neurones (donnant lieu à leur profil pharmacologique convulsivant *in vivo*), les composés de la présente invention stimulent fortement les courants produits par le GABA de la même manière que les benzodiazépines utiles thérapeutiquement.

La présente invention concerne donc les composés de formule générale : 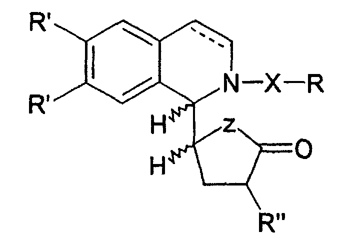 dans laquelle :
Z représente un atome de soufre, d'oxygène, NH, N-alkyle ou Nboc,
les groupes R', qui peuvent être identiques ou différents l'un de l'autre, représentent chacun un atome d'hydrogène, un groupe OCH₃ ou ensemble un groupe OCH₂O,
le groupe R" représente un hydrogène ou un groupe CH₃,
le groupe X représente un groupe carbonyle, sulfonyle ou CO₂
et le groupe R représente un groupe alkyle, aryle, alcényle ou aralkyle
ces composés pouvant se trouver sous forme de mélange racémique ou sous forme optiquement pure.

Un groupe préféré de composé selon l'invention est constitué par les composés pour lesquels Z est un atome d'oxygène ou Nboc. Les composés de la présente invention pour lesquels Z est un atome d'oxygène sont particulièrement préféré en raison de leur activité importante.

Un groupe particulier de composés selon l'invention est constitué par les composés de formule générale : 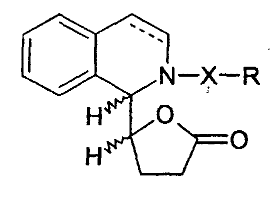 dans laquelle:
le groupe X représente un groupe carbonyle, sulfonyle ou CO₂
et le groupe R représente un groupe alkyle, aryle, alcényle ou aralkyle.

Parmi les composés préférés de l'invention, on peut citer le composé de formule : 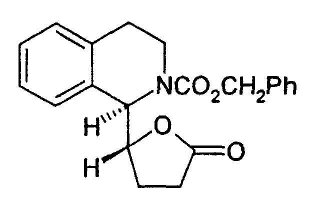 et celui de formule :

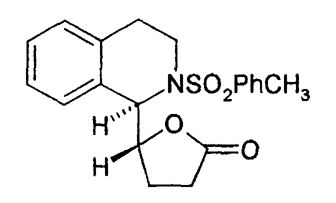

Par le terme de groupe alkyle, on entend les groupes alkyles de 1 à 4 atomes de carbones, linéaires ou ramifiés, substitués ou non substitués. Des exemples préférés de groupes alkyles sont les groupes CH₃ et C₂H₅.

Par le terme de groupe alcényle, on entend les groupes alcényles de 1 à 4 atomes de carbones, linéaires ou ramifiés, substitués ou non substitués.

Par le terme de groupes aryles on entend des cycles aromatiques ayant de 5 à 8 atomes de carbones, substitués ou non substitués, ayant un seul ou plusieurs cycle aromatiques. Les cycles aromatiques peuvent être accolés ou fusionnés. Des exemples de cycles aromatiques préférés sont les phényles, naphtyles. Des exemples de substituants préférés de ces cycles sont des groupes alkyles tel que CH₃, des atomes d'halogènes tel que Cl, des groupes alkyles halogénés tel que CF₃ ou des groupes du type OCH₃.

Par le terme de groupes aralkyles on entend des groupes aryles, définis comme ci-dessus, liés au groupe sulfonyle ou carbonyle ou CO₂ par l'intermédiaire d'un groupe alkyle défini comme précédemment. Un exemple préféré de groupe aralkyle est le groupe CH₂Ph.

Les composés selon l'invention possèdent tous un centre d'asymétrie et peuvent donc exister sous forme d'isomères optiques. La présente invention comprend aussi bien ces isomères soit séparément soit en tant que mélange.

La présente invention concerne également le mode de préparation de ces composés qui peut être le suivant :
le composé de formule générale : 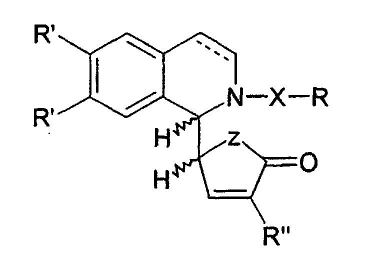
dans laquelle :
Z représente un atome de soufre, d'oxygène, NH, N-alkyle ou Nboc, de préférence un atome d'oxygène,
les groupes R', qui peuvent être identiques ou différents l'un de l'autre, représentent chacun un atome d'hydrogène, un groupe OCH₃ ou ensemble un groupe OCH₂O,
le groupe R" représente un hydrogène ou un groupe CH₃,
le groupe X représente un groupe carbonyle, sulfonyle ou CO₂
et le groupe R représente un groupe alkyle, aryle, alcényle ou aralkyle. est hydrogéné sélectivement par H₂ en présence de Pd-C pour donner les composés selon la présente invention.

Ce procédé peut comporter une étape préalable de condensation en présence d'un agent acylant ou sulfonylant d'un silyle énol éther représenté par la formule générale : 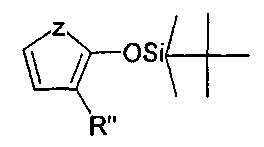 dans laquelle :
Z représente un atome de soufre, d'oxygène, NH, N-alkyle ou Nboc, de préférence un atome d'oxygène,
et R" représente un atome d'hydrogène ou un CH₃
   avec un sel de 3,4-dihydroisoquinoline représentée par la formule générale suivante: 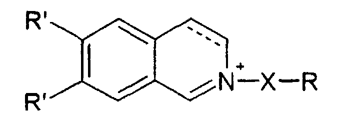
dans laquelle:
les groupes R', qui peuvent être identiques ou différents l'un de l'autre, représentent chacun un atome d'hydrogène, un groupe OCH₃ ou ensemble un groupe OCH₂O,
le groupe X représente un groupe carbonyle, sulfonyle ou CO₂
et le groupe R représente un groupe alkyle, aryle, alcényle ou aralkyle.

La présente invention concerne également les compositions pharmaceutiques comprenant à titre de principe actif un des composés définis ci-dessus et un excipient approprié. Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriés comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

La présente invention concerne également l'utilisation des ces composés comme stimulant de l'activité de l'acide γ-aminobutyrique agissant via le récepteur GABA-A du système nerveux central.

Les composés selon l'invention et les compositions pharmaceutiques les comprenant peuvent être utilisés comme médicament, en particulier pour le traitement des troubles nerveux. Ces troubles sont de préférence du type épilepsie, anxiété, dépression, troubles du sommeil et de la mémoire, attaques de panique, contractions musculaires, douleur, dépendance à l'alcool ou aux benzodiazépines ou comportements psychotiques.

### SYNTHESE

Les composés actifs de la présente invention (c'est-à-dire de structure générale **8**) peuvent tous être préparés par le même procédé (schéma 2). Brièvement, les dérivés substitués ou non-substitués de la 3,4-dihydroisoquinoline **11** (préparés selon les procédés publiés: R.L. Hillard, C.A. Parnell, K.P.C. Vollhardt, Tetrahedron, 1983, 39, 905 et W.M. Whaley, M. Meadow, J. Chem. Soc., 1953, 1067) réagissent tout d'abord dans l'acétonitrile à 0°C avec un chlorure d'acide aromatique ou un chloroformate d'aryle ou un chlorure d'arylsulfonyle pendant 15 à 30 min. Le mélange réactionnel est alors traité avec un éther diénolique de silyle **10** (préparé en faisant réagir la butyrolactone commerciale **9** avec des trialkylsilyltriflates suivant les procédés publiés : (a) G. Casiraghi, L. Colombo, G. Rassu, R. Spanu, J. Org. Chem., 1990, 55, 2565, (b) Y. Morimoto, K. Nishida, Y. Hayashi, H. Shirahama, Tet. Lett., 1993, 34, 5773, (c) G. Casiraghi, G. Rassu, P. Spanu, L. Pinna, J. Org. Chem., 1992, 57, 3760, et (d) C.W. Jefford, A.W. Sledeski, J-C. Rossier, J. Boukouvalas, Tet. Lett., 1990, 31, 5741). Après 15 à 30 minutes, le mélange réactionnel est traité pour donner le produit couplé insaturé **12** sous forme d'un mélange de 4 isomères. La double liaison du composé **12** est réduite par hydrogénation catalytique sur du palladium sur du carbone dans de l'éthanol pour donner les composés de structure générale **8**. Les deux diasteréomères de **8** (*érythro*-**8** et *thréo*-**8**) peuvent être séparés par soit a) chromatographie sur gel de silice, soit b) cristallisation fractionnelle, soit c) HPLC sur colonne de silice. Dans les cas où, dans le composé **8**, Z = N-Boc, le traitement avec de l'acide trifluoroacétique dans du dichlorométhane donne le composé déprotégé **8** (Z = NH).

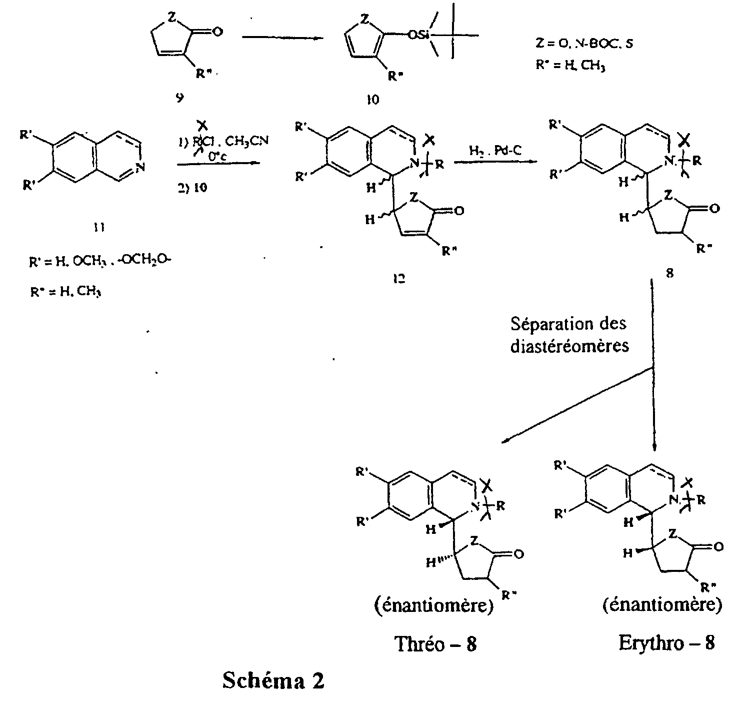

Dans le cas spécial où le groupe "R" du composé **12** est un groupe benzyloxycarbonyle ("Cbz"), alors les isomères *érythro* et *thréo* peuvent être séparés par chromatographie sur gel de silice. L'hydrogénation catalytique du composé *thréo*-**12** (l'isomère le plus actif) sur du palladium sur du carbone conduit simultanément à la réduction de la double liaison et à la suppression du groupe Cbz pour donner le composé *thréo*-**13**. La réaction de ce dernier avec un chlorure d'acide aromatique ou un chloroformate d'aryle ou un chlorure d'arylsulfonyle en présence de triéthylamine peut alors donner le composé désiré *thréo*-**8**. L'avantage de cette seconde méthode (schéma 3) pour préparer le composé *thréo*-**8** (ou *érythro*-**8**) réside dans le fait qu'elle ne requiert qu'une séparation des diasteréomères du composé **12** (XR = CO₂CH₂Ph) alors que dans la première méthode, les diastéréomères de chaque produit final **8** doivent être séparés (parfois avec difficulté).

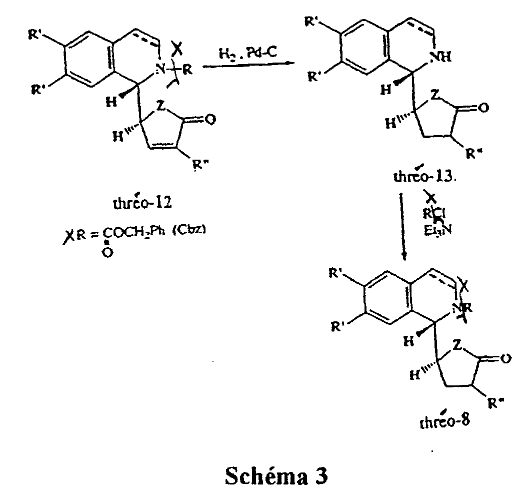

Finalement, étant donné que les composés *érythro*-**8** et *thréo*-**8** sont toujours dans un mélange d'énantiomères, ce dernier peut être convenablement séparé par HPLC sur une colonne à support chirale.

### ETUDES DE LIAISON SUR LE RECEPTEUR

Les composés synthétisés sont testés *in vitro* pour leur capacité à se lier à différents sites sur le récepteur GABA-A, incluant le site du GABA lui-même (par des études de déplacement du H³-muscimol), le site des benzodiazépines (par déplacement du H³-flunitrazépam), le site de la picrotoxine (par déplacement du S³⁵-TBPS). Brièvement, des membranes gelées provenant du cervelet ou du cerveau sans cervelet sont dégelées, centrifugées et remises en suspension dans 50 mM de tampon Tris-citrate, pH 7,4, à une concentration en protéines d'environ 1 mg/ml. Les membranes (0,5 ml) sont alors incubées dans un total de 1 ml de solution contenant 50 mM de tampon Tris-citrate, pH 7,4, 150 mM de NaCl et 2 nM de [H³]flunitrazépam ou 2 nM de [H³]muscimol en absence ou en présence de concentrations variées du composé à étudier ou de 10 µM de diazépam ou de 10 µM de GABA, pendant 90 min à 4°C, respectivement. Pour la liaison [S³⁵]TBPS, les membranes sont incubées dans un total de 1 ml de solution contenant 50 mM de tampon Tris-citrate, pH 7,4, 200 mM de NaBr et 2 nM de [S³⁵]TBPS en absence ou en présence de concentrations variées du composé à étudier ou de 10 µM de TBPS ou de picrotoxinine pendant 180 min à température ambiante (W. Sieghart, A. Schuster, Biochem. Pharmacol., 1984, 33, 4033 et J. Zezula et al., Eur. J. Pharmacol., 1996, 301, 207).

Les membranes sont alors filtrées à travers des filtres Whatman GF/B. Quand la liaison du [H³]flunitrazépam ou du [H³]muscimol est étudiée, les filtres sont rincés deux fois avec 5 ml d'une solution tampon de 50 mM de Tris-citrate glacé. Lorsque la liaison du [S³⁵]TBPS est étudiée, les filtres sont rincés trois fois avec 3,5 ml de cette solution tampon. Les filtres sont transférés dans des fioles à scintillation et soumises au comptage à scintillation après addition de 3,5 ml de fluide de scintillation. La liaison non-spécifique déterminée en présence de 10 µM de diazépam, de 10 µM de GABA ou de 10 µM de TBPS est soustraite du total de la liaison du [H³]flunitrazépam, du [H³]muscimol, ou du [S³⁵]TBPS respectivement pour obtenir la liaison spécifique.

### ETUDES ELECTROPHYSIOLOGIQUES

Les composés synthétisés sont aussi étudiés pour leur habilité à provoquer l'ouverture du canal du récepteur GABA-A ou à moduler allostériquement les courants provoqués par le GABA. Dans ce but, les récepteurs recombinants GABA-A sont exprimés dans des ovocytes de Xénope. Brièvement, les ovocytes de Xenopus laevis sont préparés, injectés, défolliculés et les courants sont enregistrés de la façon décrite (E. Sigel, J. Physiol., 1987, 386, 73 et E. Sigel, R. Baur, G. Trube, H. Möhler, P. Malherbe, Neuron, 1990, 5, 703). Les ovocytes sont injectés avec 50 ni d'ARNc dissous dans 5 mM de K-Hepes (pH 6,8). Cette solution contient les transcripts codants pour les différentes sous-unités à une concentration de 10 nM pour α₁, 10 nM pour β₂ et 50 nM pour γ₂. Les transcripts d'ARN sont synthétisés à partir de plasmides linéarisés encodant la protéine désirée en utilisant la trousse de message machine (Ambion) selon la recommandation des fabricants. Une queue poly(A) d'environ 300 résidus est ajoutée aux transcripts en utilisant le poly(A) polymérase de levure (USB ou Amersham). Les combinaisons d'ARNc sont co-precipitées dans de l'éthanol et stockées à -20°C. Les transcripts sont quantifiés sur des gels agarose après avoir été colorés avec le colorant ARN Rouge Radiant (Bio-Rad) en comparant les intensités des colorations avec différentes quantités de marqueurs de poids moléculaire (ARN-Ladder, Gibco-BRL). Les expériences électrophysiologiques sont réalisées par la méthode de pince de tension à deux électrodes à un potentiel de fixation de -80 mV. Le milieu contient 90 mM de NaCl, 1 mM de KCl, 1 mM de MgCl₂, 1 mM de CaCl₂ et 10 mM de Na-Hepes (pH 7,4). Du GABA est appliqué pendant 20 s et une période de lavage de 4 min est permise pour assurer le complet rétablissement de la désensibilisation. Le système de perfusion est nettoyé entre les applications des composés par lavage avec du sulfoxide de diméthyle pour éviter la contamination. Les composés sont appliqués à une concentration de 100 microM en absence de GABA pour voir s'ils peuvent agir comme agonistes du canal. Pour étudier la modulation allostérique, du GABA est tout d'abord appliqué seul puis en combinaison avec soit 0,1 microM soit 100 microM de composés.

### RESULTATS PHARMACOLOGIQUES

Les composés synthétisés sont testés *in vitro* pour leur habilité à déplacer le flunitrazépam tritiaté (H³-Flu, ligand sélectif du site de liaison des benzodiazépines du récepteur GABA-A), S³⁵-TBPS (sélectif pour le site de liaison de la picrotoxine) et le muscimol tritiaté (sélectif pour le site de liaison du GABA). Ces composés sont aussi testés pour leur habilité à empêcher ou à stimuler les courants provoqués par le GABA dans des ovocytes de grenouilles exprimant le sous-type α₁β₂γ₂ du récepteur du GABA.

Les résultats détaillés pour des exemples de composés sont montrés dans les tableaux 1a à 1i.

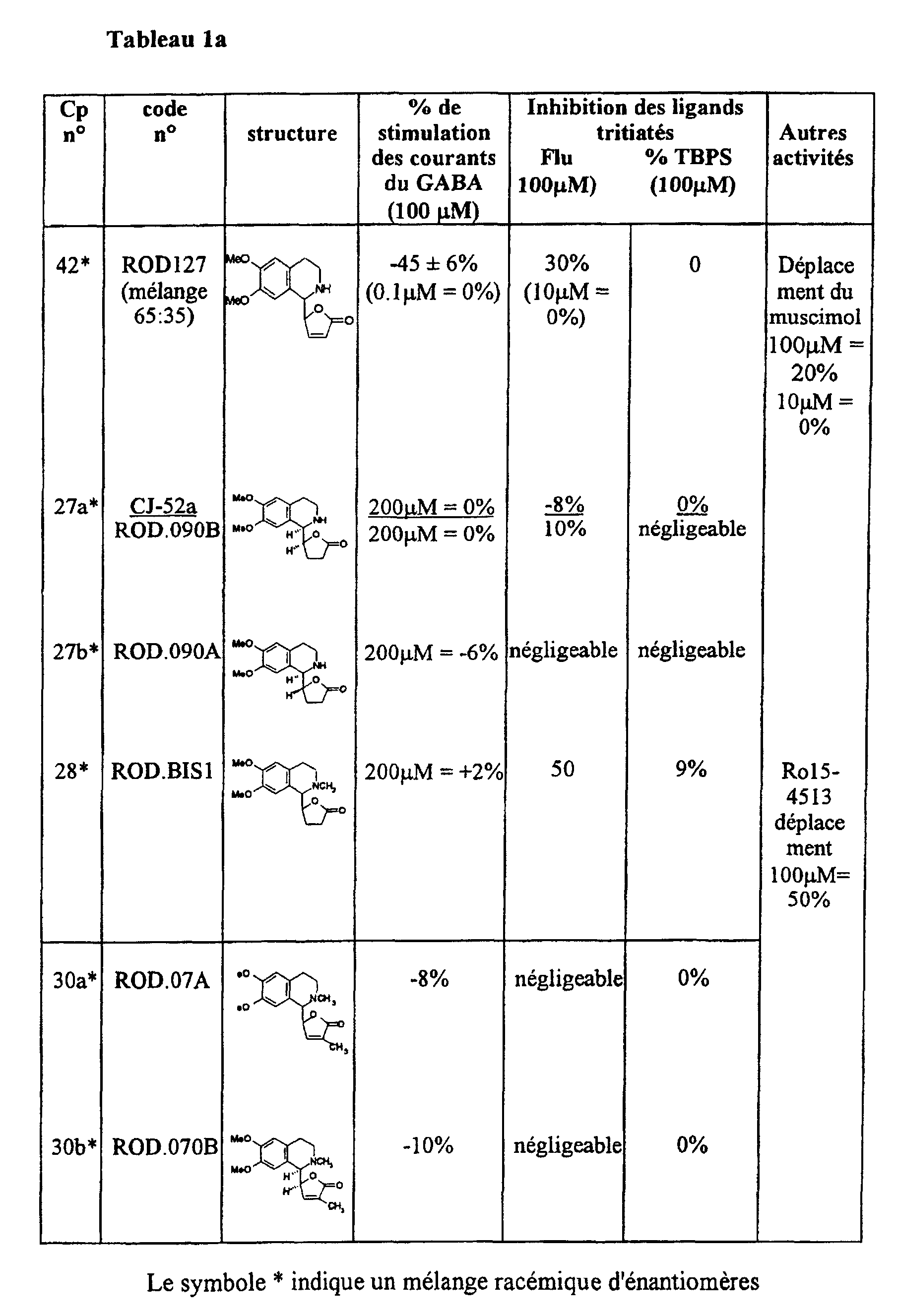

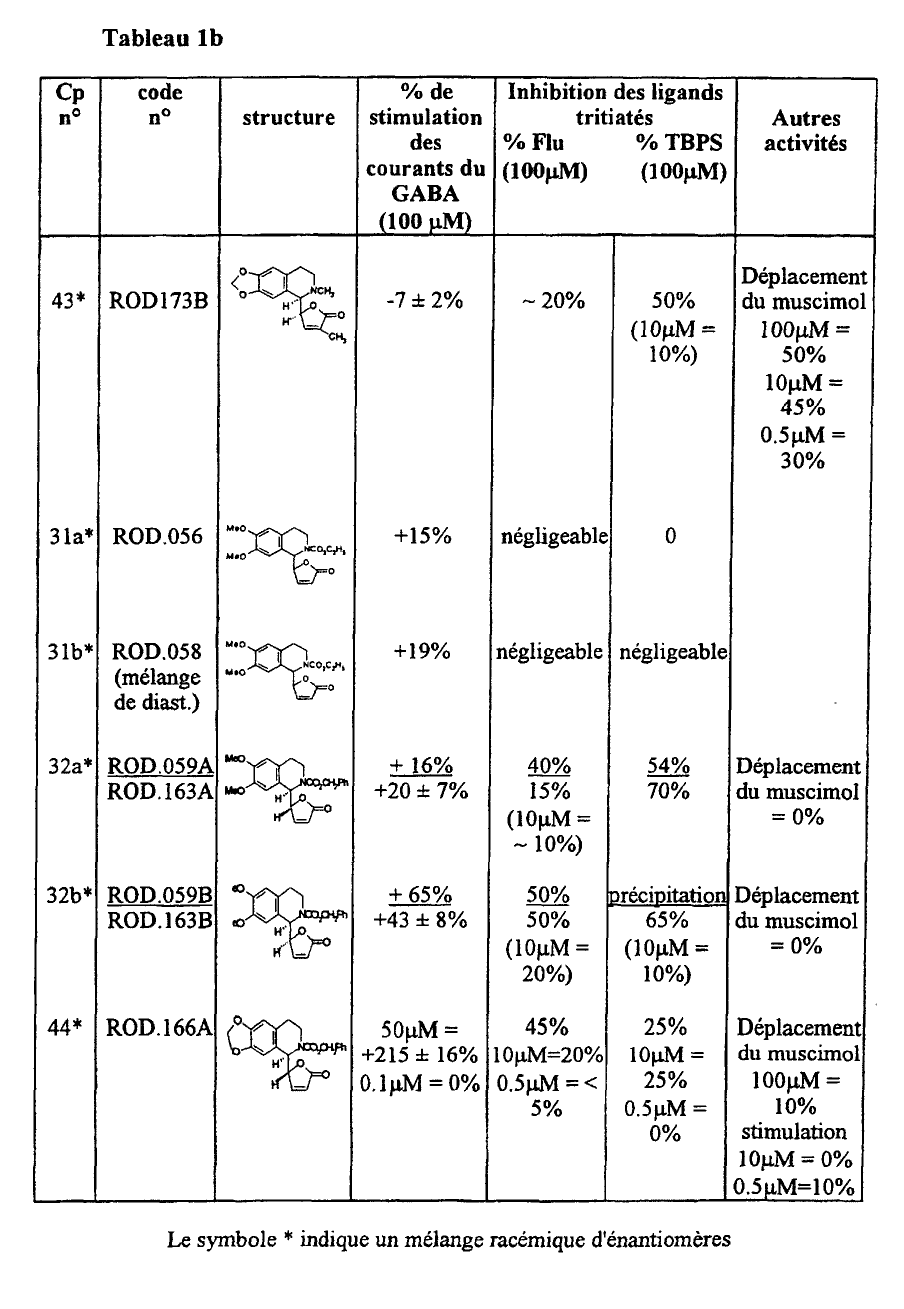

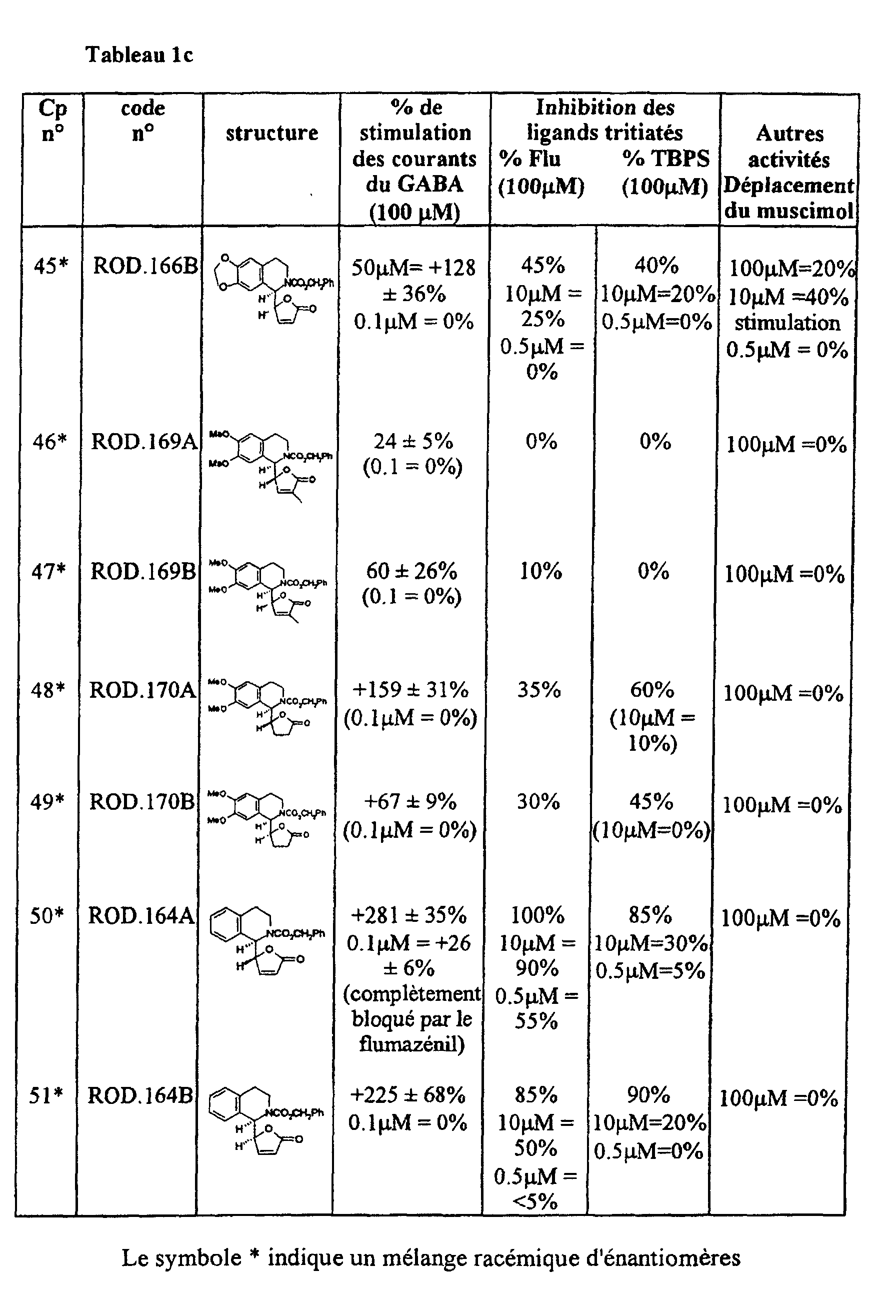

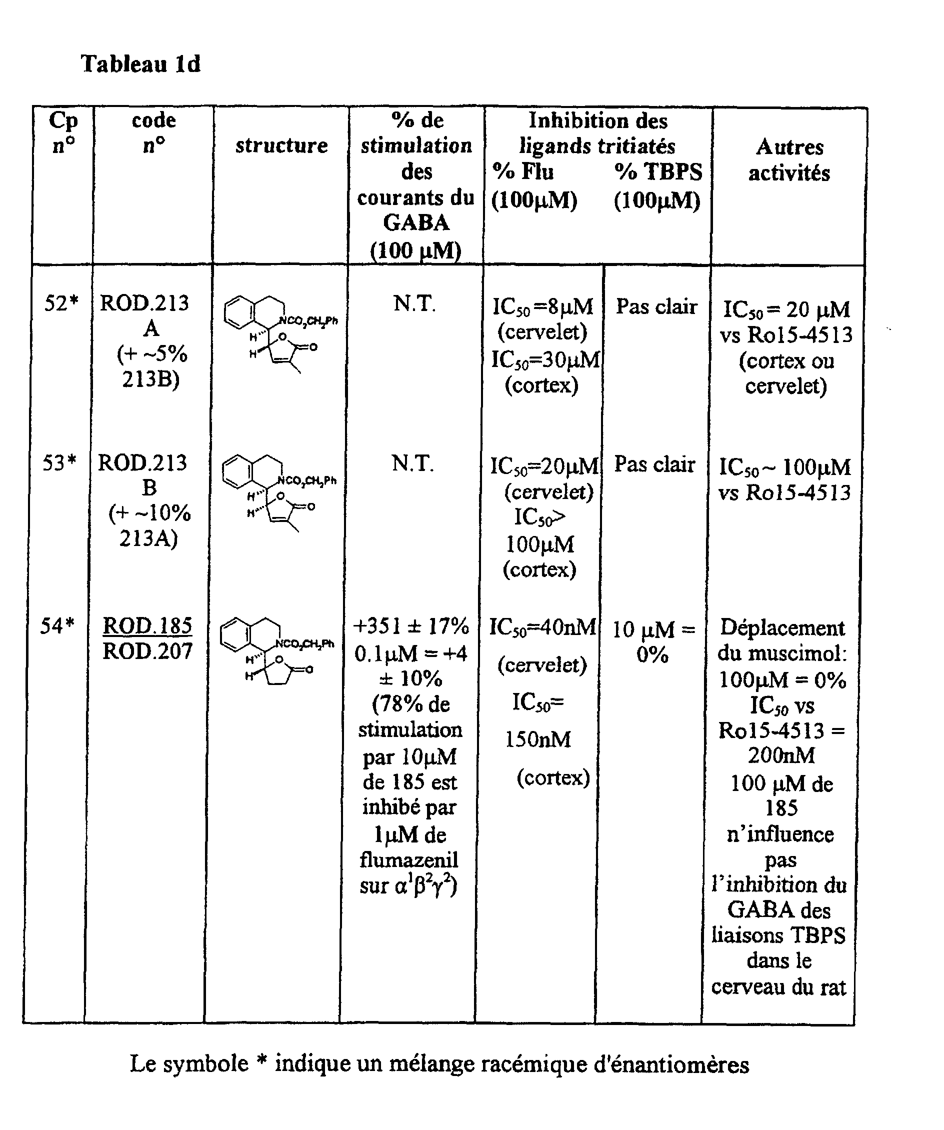

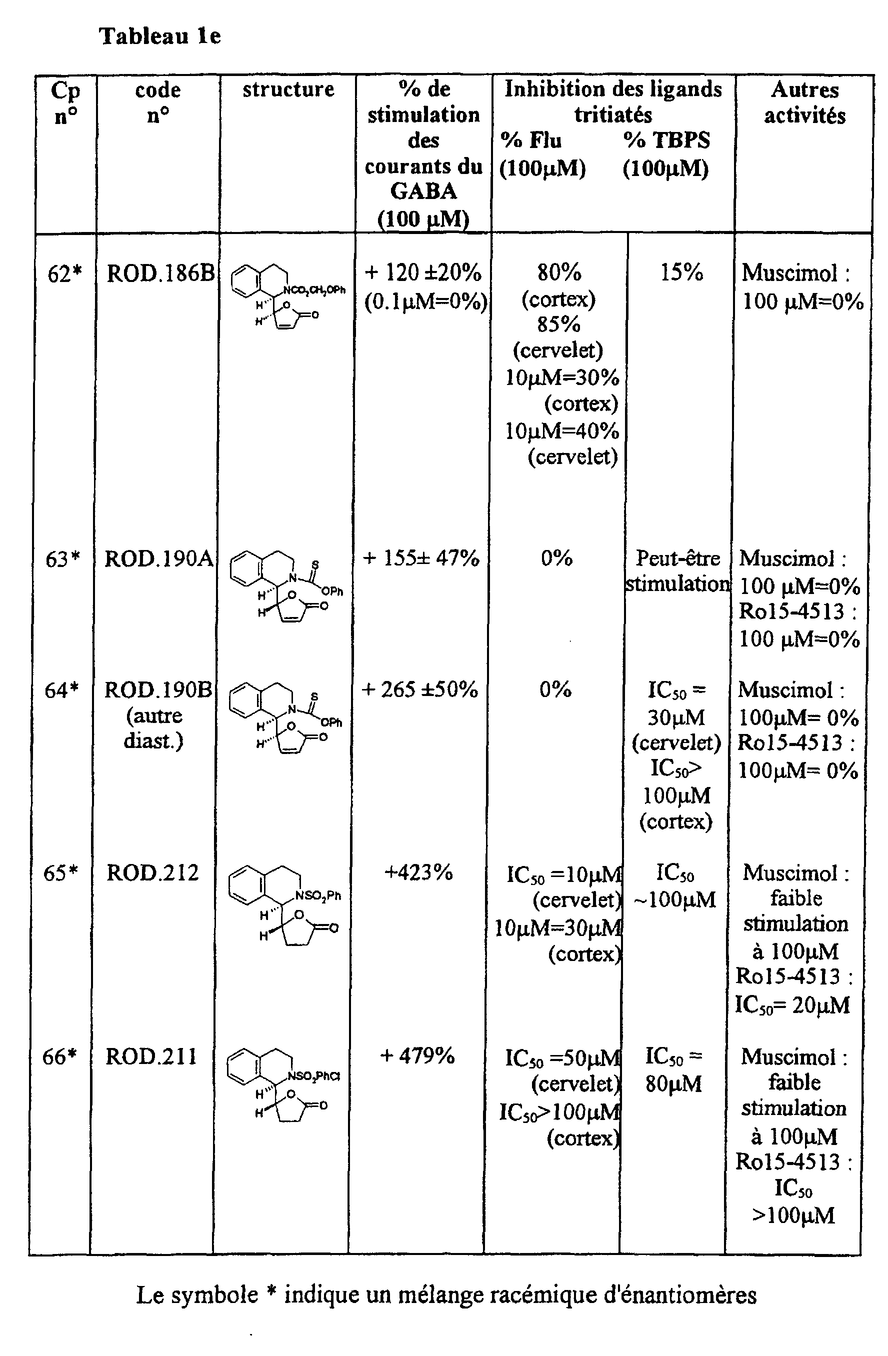

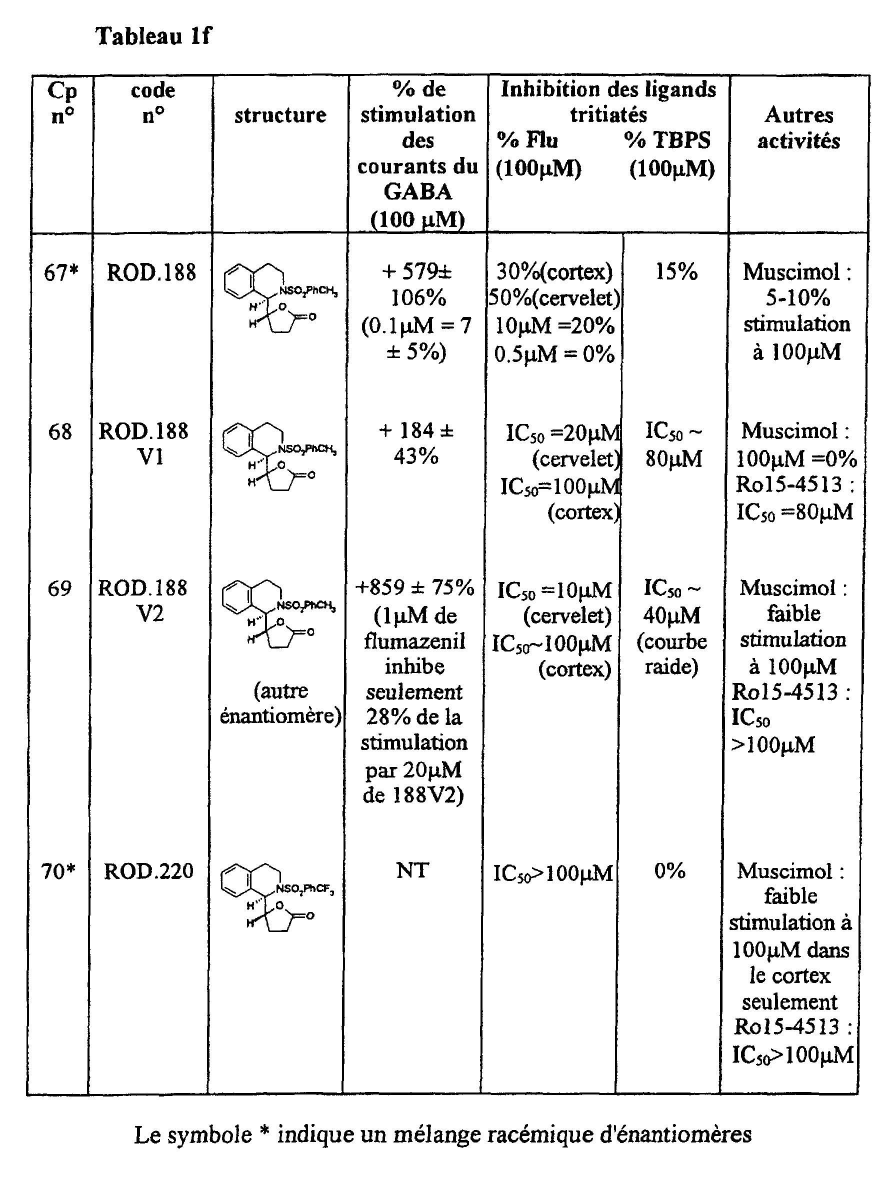

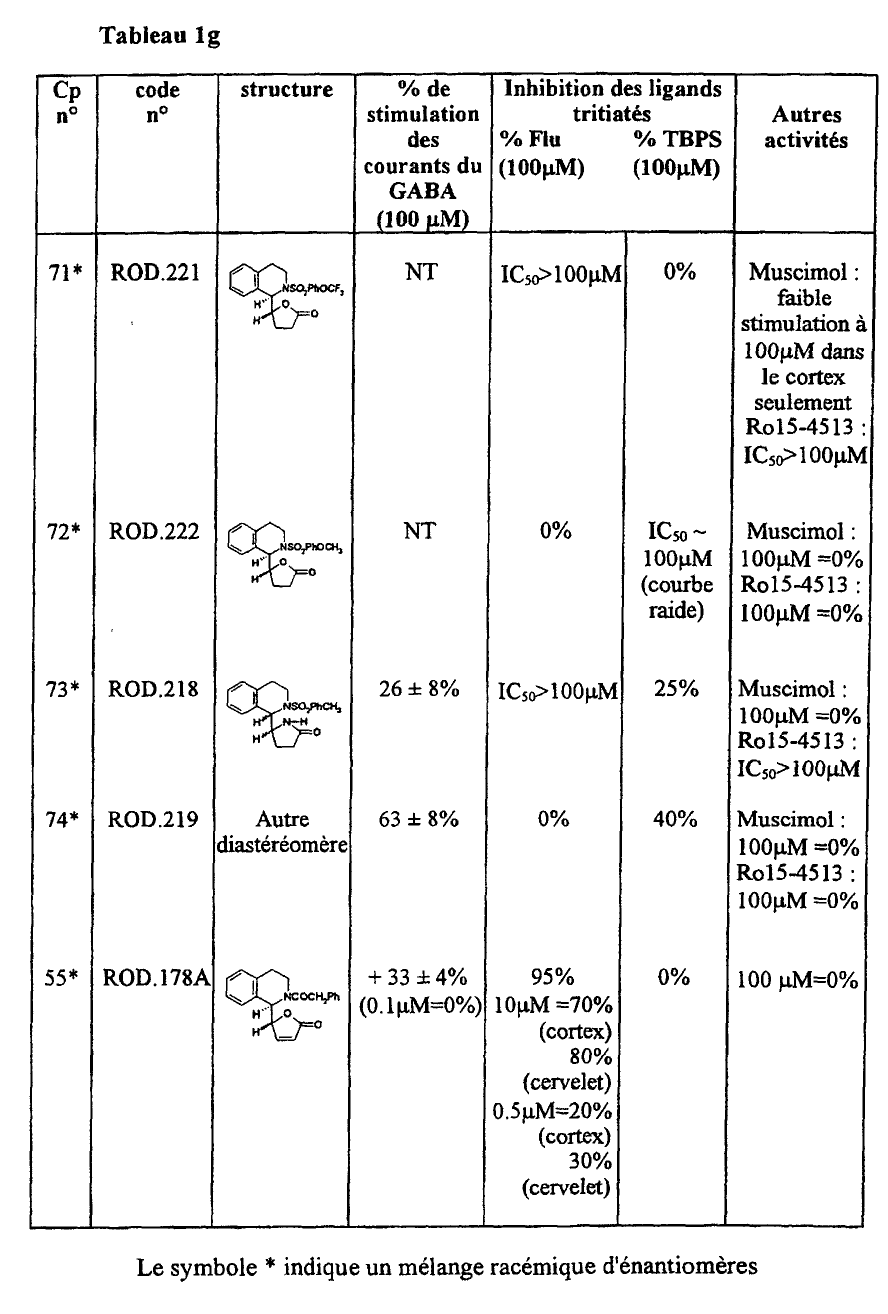

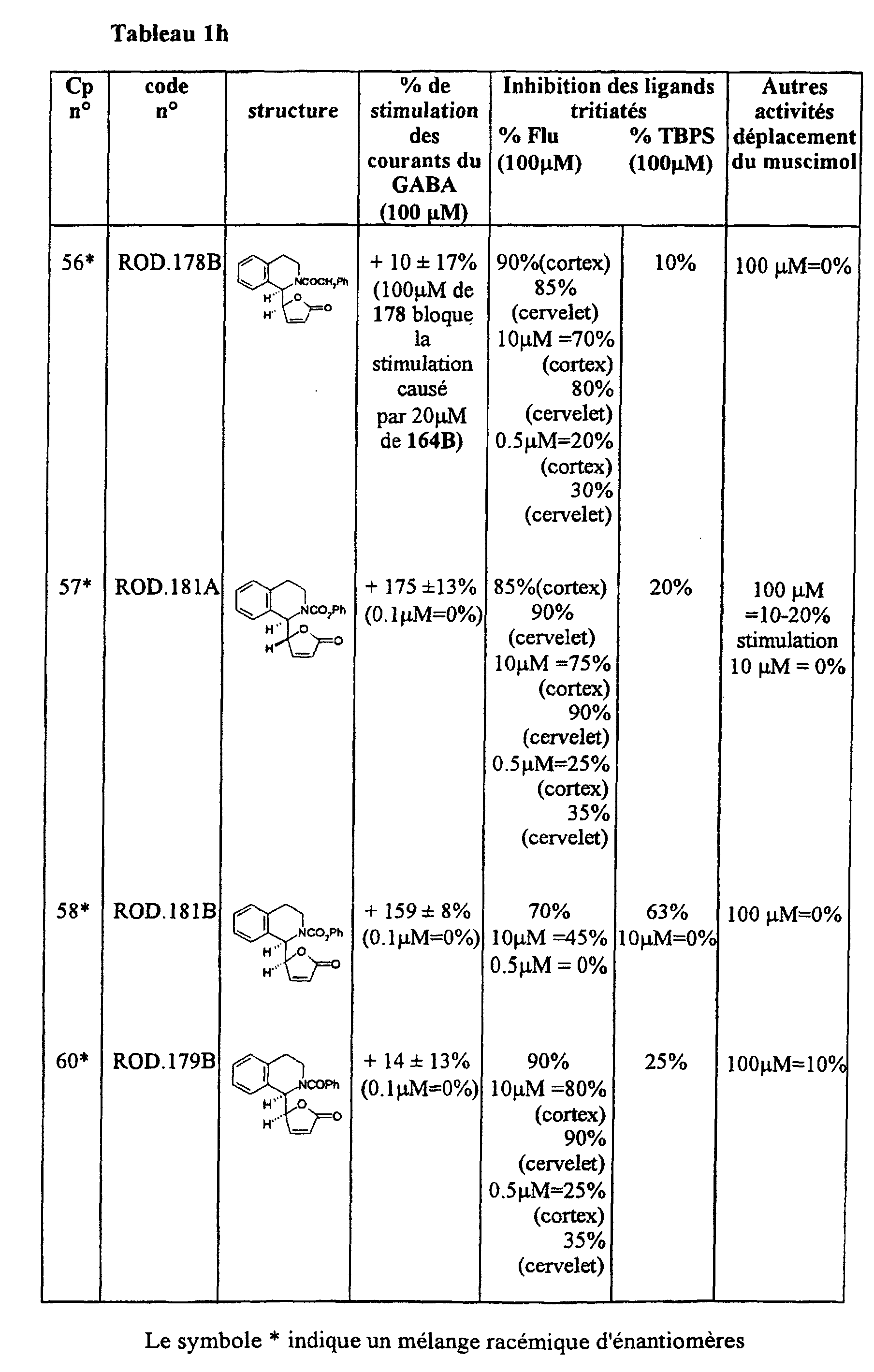

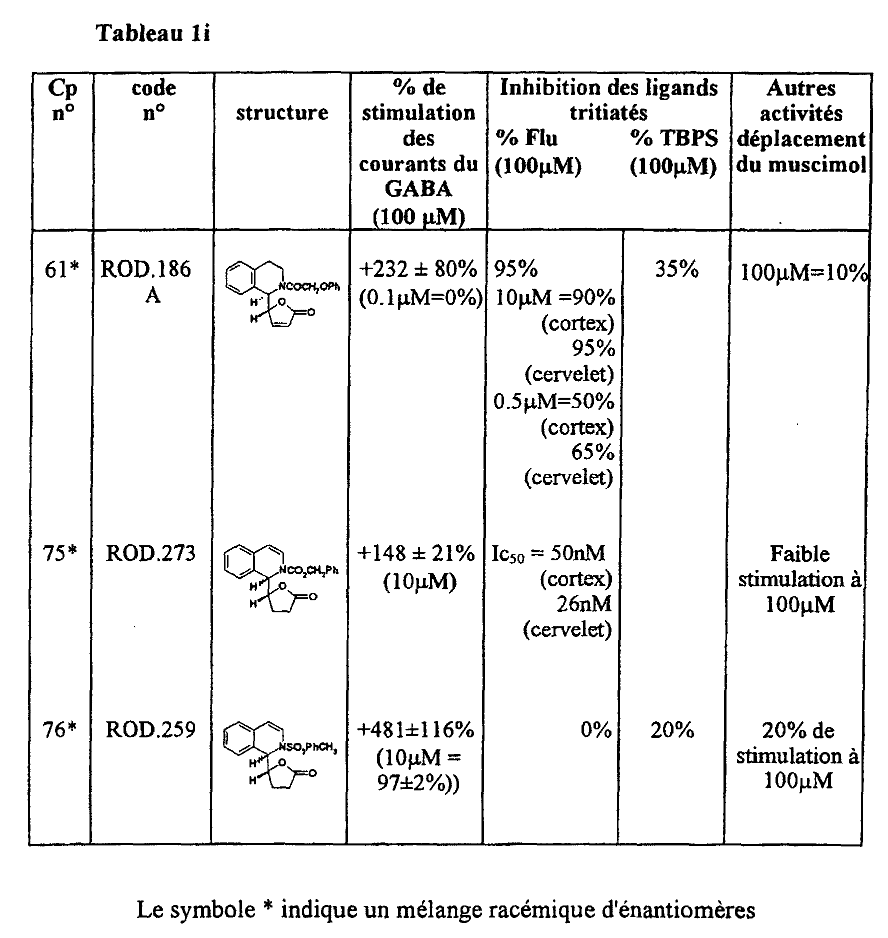

Les composés les plus actifs sont résumés dans le tableau 2 suivant.

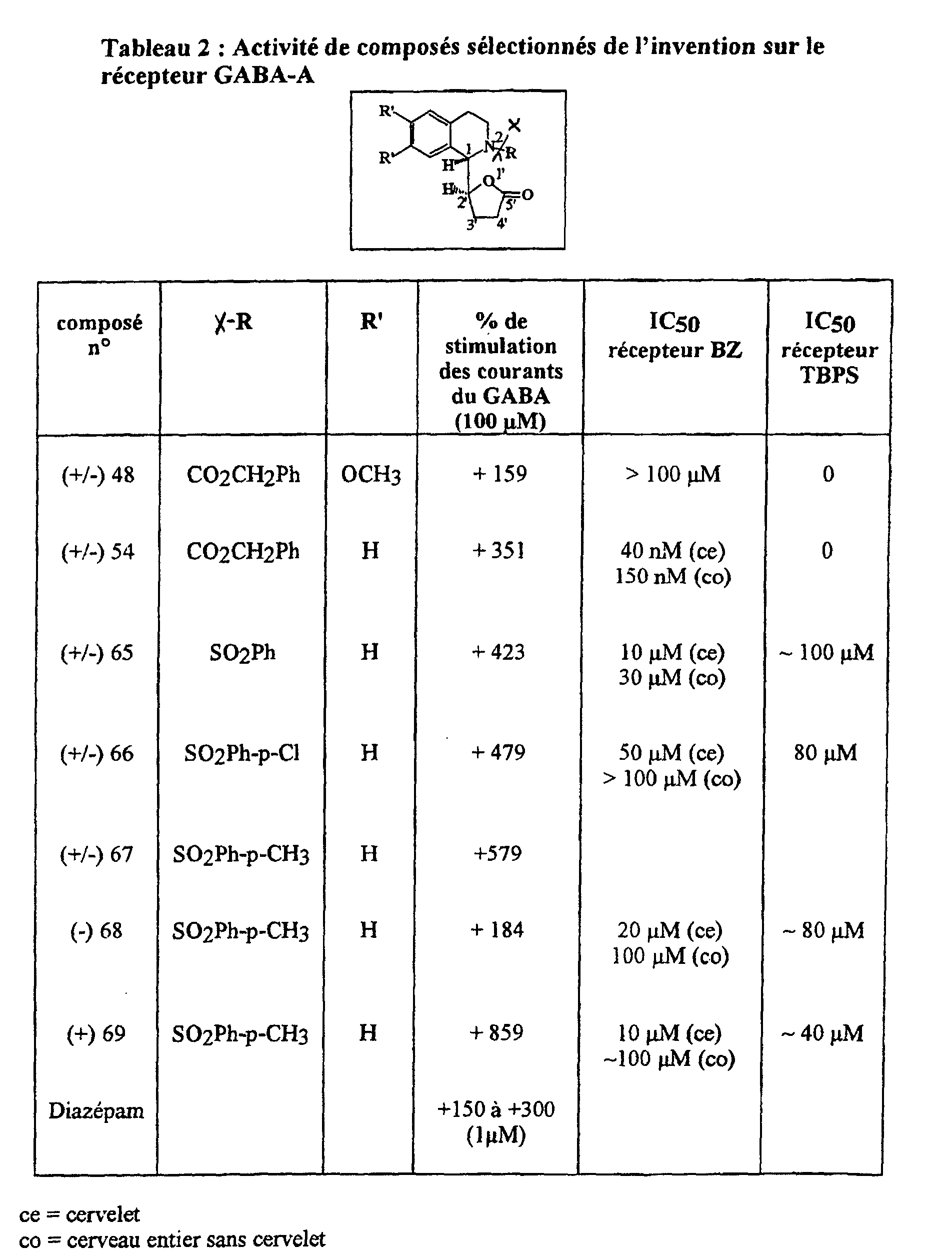

Ces exemples de composés et les résultats obtenus avec eux sont indiqués à titre non limitatif et illustrent l'invention.

Les conclusions qui peuvent être tirées de l'étude de structure-fonction sont les suivantes :

Les composés les plus actifs (c'est-à-dire ceux produisant la plus grande stimulation des courants produits par le GABA) ont :
1 - une liaison insaturée 3',4' (comparer les composés **54** et **50**) ;
2 - un atome d'oxygène à la position 1' plutôt qu'un atome d'azote (comparer les composés **67** et **73**) ;
3 - les hydrogènes à la position C-1 et C-2' ont une configuration relative "*thréo*" plutôt qu' "*érythro*" (c'est-à-dire du type de la bicuculline) (comparer les composés **48** et **49**);
4 - une rotation optique positive plutôt que négative dans le cas des purs énantiomères des diastéréomères "*thréo*" (comparer les composés **69** et **68**);
5 - aucun substituant éther d'alkyle sur le cycle "A" (comparer les composés **54** et **48**);
6 - un carbamate d'aryle ou un sulfonate d'aryle (c'est-à-dire le groupe XR) attaché à l'atome d'azote de l'isoquinoline (comparer 48 et 69 avec 55).

En ce qui concerne le mécanisme par lequel ces nouveaux composés stimulent le courant produit par le GABA, plusieurs conclusions peuvent être tirées des études de déplacement de ligand radioactif combinées avec les résultats électrophysiologiques. Tout d'abord, aucun de ces composés ne se lie avec le site de reconnaissance du GABA du récepteur GABA-A. Ainsi, aucun, ou un très faible déplacement du muscimol tritiaté est observé en présence des composés du tableau 2. De plus, en l'absence de GABA, aucun courant n'est produit dans les préparations d'ovocytes lorsque l'un quelconque de ces composés est administré.

Les composés dans lesquels le groupe "XR" est un carbamate (par exemple **54**) ou un autre substituant N-C=O (par exemple **59**, **61**) semblent stimuler les courants du GABA par liaison avec le site de reconnaissance des benzodiazépines du récepteur GABA-A de façon analogue aux benzodiazépines eux-mêmes (le diazépam par exemple). Ainsi, le composé **54** déplace le flunitrazépam tritiaté avec un IC₅₀ de 40 nM pour le type de récepteurs trouvé dans le cervelet (sous-type BZ1) et 150 nM pour les récepteurs de la partie restante du cerveau (sous-type de récepteur BZ2). Ces valeurs se comparent favorablement avec celles du diazépam, prototype des benzodiazépines, qui, toutefois, ne démontre aucune sélectivité sur les sous-types par rapport aux nouveaux composés tels que le composé **54**. La puissante stimulation des courants du GABA produite par les composés du type **54** peut être empêchée dans une large mesure par l'addition d'un antagoniste du récepteur des benzodiazépines dans la préparation d'ovocytes. Ainsi, 78% de la stimulation produite par 10 µM du composé **54** est empêchée par la co-administration de 1 µM de flumazénil. Ceci prouve que la liaison du composé **54** (et des analogues similaires) sur le récepteur des benzodiazépines est largement responsable de la stimulation des courants du GABA observée.

Un effet remarquable est observé avec les molécules dans lequel le groupe XR est un arylsulfonyle (par exemple les composés **65-69**). Dans tous les cas, les stimulations du courant sont beaucoup plus énergiques qu'avec le dérivé carbamate analogue **54**. Toutefois, les affinités de liaison avec le récepteur des benzodiazépines sont considérablement plus faibles. Ainsi, le composé **69** stimule le courant du GABA 2,5 fois plus fortement que le composé **54**, et montre pourtant une affinité de liaison 250 fois plus faible pour le récepteur des benzodiazépines. De plus, seulement 28% de l'activité stimulante du composé **69** est empêchée par le flumazénil, antagoniste du BZR. Ces résultats indiquent clairement que, de loin, la portion la plus importante (<70%) de la puissante activité stimulatrice du GABA produite par les dérivés arylsulfonyles tels que le composé **69** est due à leur interaction avec un site de liaison distinct de ceux des benzodiazépines. Bien que les dérivés arylsulfonyles déplacent aussi faiblement le TBPS de son site de liaison sur le récepteur GABA-A, il n'y a pas de corrélation observable entre ces affinités de liaison et les activités stimulantes du GABA (Tableau 2).

D'autres expériences démontrent que bien que le composé **69** ne produise pas des courants par lui-même, il peut augmenter les courants produits par le pentobarbital. A l'opposé, les courants produits par le GABA stimulés par le pentobarbital ne sont pas davantage stimulés par le composé **69**. De plus, un récepteur muté incapable de répondre à une application de loreclézole montre une stimulation non altérée du courant par le composé **69**. Finalement, les récepteurs recombinants de la composition de sous-unité αₓβ₂γ₂ dans laquelle x = 1, 2, 3, 5, 6 sont aussi testés pour la stimulation par le composé **69** du courant du GABA. La plus importante stimulation du courant est observée pour x = 6.

On peut donc conclure qu'alors que les composés carbamate tels que le **54** représentent une nouvelle et une sous-classe sélective de ligands agonistes du récepteur des benzodiazépines, les analogues arylsulfonyles, bien que démontrant le même type d'activité stimulatrice du GABA que les benzodiazépines, ne font ceci qu'en se liant principalement sur un site encore non caractérisé du récepteur GABA-A. Ces composés sont donc nouveaux à la fois d'un point de vue structurel et aussi du point de vue de leur nouveau mode d'action. Les activités stimulatrices du GABA de ces composés sont de portée thérapeutique, ce qui est démontré par le fait que les benzodiazépines sont largement prescrites. Le nouveau mode d'action apparent des composés de la présente invention suggère qu'ils peuvent posséder les effets cliniques bénéfiques des benzodiazépines mais avec de plus faibles ou même pas d'effets secondaires.

Il a été noté de façon surprenante que les composés possédant une double liaison dans la partie isoquinoline (composés **75** et **76**) sont particulièrement actifs.

De plus, certains de ces produits démontrent une sélectivité pour l'une ou l'autre des multiples sous-types du récepteur GABA-A. Ainsi bien que le composé **32a** (Tableau 1b) ne stimule que faiblement le récepteur de type α1β2γ2, cette stimulation est fortement augmentée dans les cas où la sous-unité α1 est remplacée par la sous-unité α2, α3, α5 ou α6. De même, le composé **73** (Tableau 1g) ne stimule que faiblement le récepteur α1β2γ2 mais en présence de récepteurs possédant une sous-unité α5, une inhibition ( au lieu d'une stimulation) des courants gabaergiques est observée. Ce résultat montre que 73 ainsi que les autres molécules analogues de la présente invention ont un effet bénéfique sur la mémoire.

Les exemples suivants donnés à titre non limitatif, illustrent l'invention.

### PROCEDE DE SYNTHESE

### Le 2-(tert-Butyldiméthylsilyloxy)furane (10, Z = O).

A une solution fraîchement distillée de la 2(5H)-furanone (2,86 g, 34 mmol) dans du dichlorométhane (20 ml), on ajoute de la triéthylamine distillée (6,6 ml, 47,4 mmol) sous argon à 0°C suivi ensuite rapidement par du sulfonate de tert-butyldiméthylsilyl trifluorométhane (8,6 ml, 37,5 mmol). On permet au mélange réactionnel de revenir à température ambiante et l'agitation est maintenue pendant deux heures. Le solvant est alors enlevé sous pression réduite et le résidu huileux est purifié par chromatographie flash sur gel de silice (acétate d'éthyle-heptane, 1:9) pour obtenir ainsi le composé **10** (Z = O) sous forme d'une huile légèrement colorée (5,3 g, 79%). Les données spectroscopiques de ce composé (RMN H¹ et C¹³) sont identiques à celles décrites dans la littérature (G. Rassu, F. Zanardi, L. Battistini, E. Gaetani, G. Casiraghi, J. Med. Chem., 1997, 40, 168).

### Les (±)-(1R,2'R)- et (±)-(1R,2'S)-1-(2,5-dihydro-5-oxo-2-furyl)-2-N-benzyloxycarbonyl-6,7-diméthoxy-1,2,3,4-tétrahydroisoqumoline (respectivement 32a et 32b).

A une solution de 3,4-dihydro-6,7-diméthoxyisoquinoline (960 mg, 5 mmol) (R.L. Hillard, C.A. Parnell, K.P.C. Vollhardt, Tetrahedron, 1983, 39, 905 et W.M. Whaley, M. Meadow, J. Chem. Soc., 1953, 1067) dans de l'acétonitrile anhydre (10 ml), on ajoute lentement du chloroformate de benzyle à 0°C sous argon (0,75 ml, 5,25 mmol). Après la fin de l'addition, on permet au mélange réactionnel de couleur rouge foncé de revenir à la température ambiante et l'agitation est maintenue pendant 15 minutes. Une solution de 2-(tert-butyldiméthylsilyloxy)furane (**10**, Z=O) (1 g, 5 mmol) dans de l'acétonitrile (1 ml) est alors ajoutée lentement et le mélange réactionnel est agité pendant 15 minutes à température ambiante. La solution est neutralisée avec une solution aqueuse à 5 % d'hydrogénocarbonate de sodium (40 ml) et le mélange est extrait avec du dichlorométhane (3 x 30 ml). Les extraits organiques réunis sont séchés sur MgSO₄ et les solvants sont enlevés sous pression réduite laissant un produit brut qui est purifié par colonne chromatographique sur gel de silice (acétate d'éthyle-heptane, 7:3). L'analyse RMN H¹ du produit purifié (1,3 g, 63 %) indique la présence d'un mélange (3 :2) de diastéréomères.
Les diastéréomères sont séparés par HPLC préparatif sur une colonne de gel de silice PrépaPak (15-20 µM) en utilisant de l'acétate d'éthyle-heptane (35:65) comme éluant, une vitesse d'écoulement de 50 ml/min et une pression de 220 psi. Le premier composé à être élué est l'isomère majeur *thréo* (**32a**, temps de rétention : 10,3 min) qui est cristallisé dans du méthanol et a un point de fusion de 143°C. Analyse élémentaire pour C₂₃H₂₃NO₆.0,25H₂O : Calculée, % : C, 66,74 ; H, 5,72; N, 3,38. Trouvée, % : C, 66,73 ; H, 5,61; N, 3,42. Le diastéréomère mineur **(32b)** est élué en 12,1 min et est cristallisé dans du méthanol. Point de fusion : 154°C.
Analyse élémentaire pour C₂₃H₂₃NO₆.0,3SH₂O : Calculée, % : C, 66,45 ; H, 5,75 ; N, 3,37. Trouvée, % C, 66,47; H, 5,85 ; N, 3,13.

### La (±)-érythro-6,7-Diméthoxy-1-(2,3,4,5-tétrahydro-5-oxo-2-furyl)-1,2,3,4-tétrahydroisoquinoline (27a).

Une solution de composé *érythro* **32b** (100 mg, 0,24 mmol) dans de l'éthanol (8 ml) est hydrogénée à pression atmosphérique pendant deux heures en présence de 10 % de palladium sur du carbone (20 mg). Le catalyseur est enlevé par filtration et lavé copieusement avec de l'éthanol. Le filtrat et les eaux de lavage réunis sont évaporés sous pression réduite et le résidu est purifié par chromatographie flash sur gel de silice (150 mbar) en utilisant le dichlorométhane-éthanol (95:5) comme éluent. Le composé **27a** est obtenu sous forme d'un solide de couleur blanc cassé (39 mg, 58%) ayant un point de fusion de 119°C.
Analyse élémentaire pour C₁₅H₁₉NO₄.0,4H₂O : Calculée, % : C, 63,32 ; H, 7,01 ; N, 4,92. Trouvée, % : C, 63,24 ; H, 6,91 ; N, 4,65.

### La (±)-thréo-6,7-Diméthoxy-1-(2,3,4,5-tétrahydro-5-oxo-2-furyl)-1,2,3,4-tétrahydroisoquinoline (27b).

En suivant le même procédé que pour la préparation du composé **27a,** l'hydrogénation de l'isomère *thréo* **32a** (100 mg, 0,24 mmol) permet d'obtenir le composé **27b** sous forme d'une huile jaune (39 mg, 58%).
Analyse élémentaire pour C₁₅H₁₉NO₄.0,6H₂O : Calculée, % : C, 62,53 ; H, 7,07 ; N, 4,86. Trouvée, % : C, 62,33 ; H, 6,76 ; N, 4,77.

### La (±)--(1R,2'R)-2-N-Benzyloxycarbonyl-6,7-diméthoxy-1-(2,3,4,5-tétrahydro-5-oxo-2-furyl)-1,2,3,4-tétrahydroisoquinoline (48).

A une solution du dérivé *thréo* **32a** (50 mg, 0,12 mmol) et d'héxahydrate de chlorure de nickel (II) (3 mg, 0,012 mmol) dans du THF (0,8 ml) et du méthanol (0,2 ml) maintenue à 0°C, on ajoute du borohydrure de sodium (9,25 mg, 0,24 mmol) par portion pendant 15 minutes. On permet au mélange réactionnel de revenir à la température ambiante et après 15 minutes d'agitation le mélange est refroidi à nouveau jusqu'à 0°C, on ajoute de l'eau (5 ml) et la solution est amenée au pH 1 par addition de 3N d'HCl. Le mélange est extrait avec du dichlorométhane (3 x 5 ml), les extraits organiques réunis sont lavés successivement par de l'hydrogénocarbonate de sodium aqueux saturé (5 ml) et du chlorure de sodium aqueux saturé (5 ml) et séché sur MgSO₄. Le solvant est enlevé sous pression réduite laissant un résidu qui est purifié par colonne chromatographique sur gel de silice (acétate d'éthyle-heptane 7:3) pour obtenir le composé **48** sous forme d'un solide amorphe blanc (40 mg, 80%).
Analyse élémentaire pour C₂₃H₂₅NO₆.0,2H₂O : Calculée, % : C, 66,56 ; H, 6,17 ; N, 3,37. Trouvée, % : C, 66,48 ; H, 6,25 ; N, 3,32.

### La (±)-(1R,2'R)-2-N-Benzyloxycarbonyl-1-(2,3,4,5-tétrahydro-5-oxo-2-furyl)-1,2-dihydroisoquinoline (75)

On utilise le même procédé qui a servi pour la préparation des composés **32a** et **32b**, sauf que le produit de départ est l'isoquinoline. Le produit de couplage (seul l'isomère 1R,2'R est formé) est obtenu avec un rendement de 76%. La liaison double de la fonction furanone est réduite sélectivement par hydrogénation à un atmosphère dans l'acétate d'éthyle en présence du catalyseur de Lindlar. Le composé **75,** purifié sur colonne de silice, est obtenu avec un rendement de 90%. Son point de fusion est de 109-11°C.
SM(IC) = 350 (MH+)

### La (±)-(1R,2'R)-2-N-Tosyl-1-(2,3,4,5-tétrahydro-5-oxo-2-furyl)-1,2-dihydroisoquinoline (76)

Le même procédé qui a servi pour la préparation du composé **75** est utilisé sauf que le chlorure de p-toluènesulfonyle est utilisé à la place du chloroformiate de benzyle lors du couplage. Le composé **76** est obtenu avec une rendement global de 60% pour les deux étapes (couplage et hydrogénation). Son point de fusion est de 147-149 °C.
SM (IC) : 370 (MH+).

### Procédé général pour la N-acylation et la N-sulfonylation de la (±)-thréo-1-(2,3,4,5-tétrahydro-5-oxo-2-furyl)-1,2,3,4-tétrahydroisoquinoline.

Une solution du composé recherché (50 mg, 0,23 mmol) (préparé de la même façon que le composé **27a** mais à partir du composé **50**) dans du dichlorométhane anhydre (2 ml) est traitée sous argon et à température ambiante avec la triéthylamine (39 µl, 0,28 mmol) et l'électrophile approprié RCl (1,2 eq). Le mélange réactionnel est agité pendant la nuit, du dichiorométhane (20 ml) est ajouté et la solution est lavée avec de l'hydrogénocarbonate de sodium aqueux saturé (20 ml). La phase aqueuse est extraite avec du dichlorométhane (2 x 10 ml), les phases organiques sont réunies, séchées sur MgSO₄ et le solvant est enlevé sous pression réduite. Le résidu résultant est purifié par chromatographie flash sur gel de silice (150 mbar) en utilisant le système de solvant indiqué ci-dessous.

Les composés suivants sont préparés de cette manière :
*La (±)-(1R,2'R)-1-(2,3,4,5-Tétrahydro-5-oxo-2-furyl)-2-N-p-toluènesulfonyl-1,2,3,4-tétrahydro-isoquinoline (**67**) est préparée en utilisant le* chlorure de p-toluènesulfonyle comme électrophile et l'acétate d'éthyle-heptane (2:3) comme éluant de colonne. Le composé **67** est obtenu sous forme d'un solide blanc avec un rendement de 85% et un point de fusion de 140-142°C.
   Analyse élémentaire pour C₂₀H₂₁NSO₄ : Calculée, %.: C, 64,67; H, 5,66; N, 3,77 ; S, 8,63. Trouvée, % : C, 64,53 ; H, 5,67 ; N, 4,06 ; S, 8,51.
*La (±)-(1R,2'R)-1-(2,3,4,5-Tétrahydro-5-oxo-2-furyl)-2-N-p-chlorobenzènesulfonyl-1,2,3,4-tétrahydro-isoquinoline (****66****)* est préparée en utilisant le chlorure de p-chlorobenzènesulfonyle comme électrophile et l'acétate d'éthyle-heptane (2:3) comme éluant de colonne. Le composé **66** est obtenu avec un rendement de 82 % sous forme d'un solide blanc ayant un point de fusion de 145-147°C.
   Analyse élémentaire pour C₁₉H₁₈NSO₄Cl :
   Calculée, % : C, 58,18 ; H, 4,59 ; N, 3,57 ; S, 8,16.
   Trouvée, % : C, 57,97 ; H, 4,74 ; N, 3,41; S, 7,92.
*La (±)-(1R,2'R)-2-N-Benzènesulfonyl-1-(2,3,4,5-tétrahydro-5-oxo-2-furyl)-1,2,3,4-tétrahydro-isoquinoline (****65****)* est préparée en utilisant le chlorure de benzènesulfonyle comme électrophile et l'acétate d'éthyle-heptane (2:3) comme éluant de colonne. Le composé **65** est obtenu avec un rendement de 81% sous forme d'un solide blanc ayant un point de fusion de 177-179°C.
   Analyse élémentaire pour C₁₉H₁₉NSO₄.0,4 H₂O : Calculée, % : C, 62,59 ; H, 5,47 ; N, 3,84 ; S, 8,79. Trouvée, % : C, 62,54 ; H, 5,38 ; N, 3,92 ; S, 8,71.
*La (±)-(1R,2'R)-2-N-Benzyloxycarbonyl-1-(2,3,4,5-tétrahydro-5-oxo-2-furyl)-1,2,3,4-tétrahydro-isoquinoline (****54****)* est préparée en utilisant le chloroformate de benzyle comme électrophile, un temps de réaction de 4 heures et l'acétate d'éthyle-heptane (1:1) comme éluant de colonne. Le composé **54** est obtenu avec un rendement de 83% sous forme d'une huile incolore qui se solidifie à la longue lorsqu'elle repose et ayant un point de fusion de 120-122°C.
   Analyse élémentaire pour C₂₁H₂₁NO₄.0,2 H₂O : Calculée, % : C, 71,05 ; H, 6,08 ; N, 3,95. Trouvée, % : C, 71,09 ; H, 6,09 ; N, 3,76.

## Revendications

1. Composé représenté par la formule générale suivante : 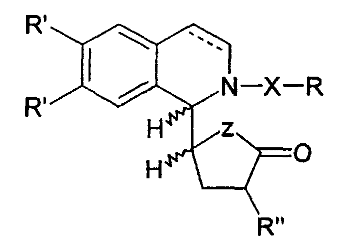 dans laquelle :
Z représente un atome de soufre, d'oxygène, NH, N-alkyle ou Nboc,
les groupes R', qui peuvent être identiques ou différents l'un de l'autre, représentent chacun un atome d'hydrogène, un groupe OCH₃ ou ensemble un groupe OCH₂O,
le groupe R" représente un hydrogène ou un groupe CH₃,
le groupe X représente un groupe carbonyle, sulfonyle ou CO₂,
et le groupe R représente un groupe alkyle, aryle, alcényle ou aralkyle
sous forme de leur mélange racémique ou de leurs isomères optiquement purs.

2. Composé selon la revendication 1 **caractérisé en ce que** Z représente un atome d'oxygène ou Nboc.

3. Composé selon les revendications 1 et 2 **caractérisé en ce que** Z est un atome d'oxygène.

4. Composé selon les revendications 1 à 3 **caractérisé en ce qu'**il est représenté par la formule générale : 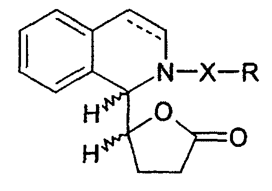 dans laquelle :
le groupe X représente un groupe carbonyle, sulfonyle ou CO₂
et le groupe R représente un groupe alkyle, aryle, alcényle ou aralkyle.

5. Composé selon les revendications 1 à 4 **caractérisé en ce qu'**il est représenté par la formule :

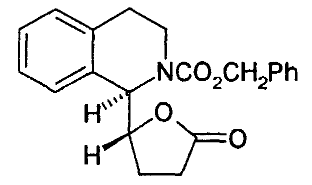

6. Composé selon les revendications 1 à 4 **caractérisé en ce qu'**il est représenté par la formule :

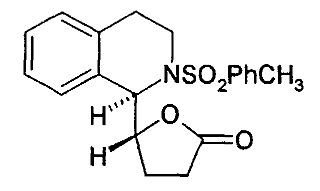

7. Procédé de préparation des composés selon les revendications 1 à 6 **caractérisé en ce que** le composé de formule générale : 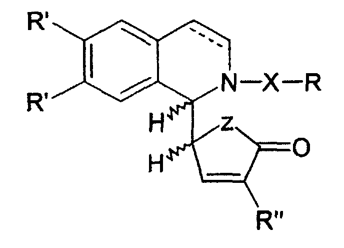 dans laquelle :
Z représente un atome de soufre, d'oxygène, NH, N-alkyle ou Nboc,
les groupes R', qui peuvent être identiques ou différents l'un de l'autre, représentent chacun un atome d'hydrogène, un groupe OCH₃ ou ensemble un groupe OCH₂O,
le groupe R" représente un hydrogène ou un groupe CH₃,
le groupe X représente un groupe carbonyle, sulfonyle ou CO₂
et le groupe R représente un groupe alkyle, aryle, alcényle ou aralkyle. est hydrogéné sélectivement par H₂ en présence de Pd-C pour donner le composé selon la revendication 1.

8. Procédé de préparation selon la revendication 7 **caractérisé en ce qu'**il comporte une étape préalable de condensation en présence d'un agent acylant ou sulfonylant d'un silyle énol éther représenté par la formule générale : 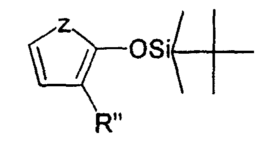 dans laquelle :
Z représente un atome de soufre, d'oxygène, NH, N-alkyle ou Nboc
et R" représente un atome d'hydrogène ou un CH₃ avec un sel de 3,4-dihydroisoquinoline représentée par la formule générale suivante: 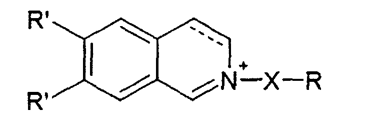
dans laquelle :
les groupes R', qui peuvent être identiques ou différents l'un de l'autre, représentent chacun un atome d'hydrogène, un groupe OCH₃ ou ensemble un groupe OCH₂O,
le groupe X représente un groupe carbonyle, sulfonyle ou CO₂
et le groupe R représente un groupe alkyle, aryle, alcényle ou aralkyle.

9. Composition pharmaceutique comprenant un composé selon les revendications 1 à 6 et un support pharmaceutique approprié.

10. Composé selon les revendications 1 à 6 et 9 pour son utilisation comme stimulant de l'activité de l'acide γ-aminobutyrique agissant via le récepteur GABA-A du système nerveux central.

11. Composé selon les revendications 1 à 6 et 9 pour son utilisation comme médicament.

12. Utilisation des composés selon les revendications 1 à 6 et 9 pour la fabrication d'un médicament destiné au traitement des troubles nerveux.

13. Utilisation selon la revendication 12 **caractérisée en ce que** les troubles nerveux sont du type épilepsie, anxiété, dépression, troubles du sommeil et de la mémoire, attaques de panique, contractions musculaires, douleur, dépendance à l'alcool ou aux benzodiazépines ou comportements psychotiques.

## Patentansprüche

1. Verbindung, die durch die folgende allgemeine Formel: 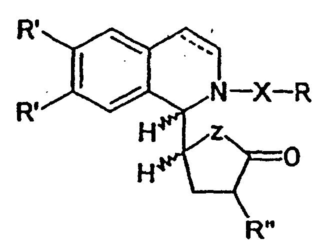 dargestellt wird, in der:
Z ein Schwefel-, Sauerstoffatom, NH, N-Alkyl oder Nboc darstellt,
die Gruppen R', die identisch oder voneinander verschieden sein können, jeweils ein Wasserstoffatom, eine OCH₃-Gruppe oder zusammen eine OCH₂O-Gruppe darstellen,
die Gruppe R" einen Wasserstoff oder eine CH₃-Gruppe darstellt,
die Gruppe X eine Carbonyl-, Sulfonyl- oder CO₂-Gruppe darstellt und
die Gruppe R eine Alkyl-, Aryl-, Alkenyl- oder Aralkyl-Gruppe darstellt,
in Form ihrer racemischen Mischung oder ihrer optisch reinen Isomeren.

2. Verbindung nach Anspruch 1, **dadurch gekennzeichnet, dass** Z ein Sauerstoffatom oder Nboc darstellt.

3. Verbindung nach den Ansprüchen 1 und 2, **dadurch gekennzeichnet, dass** Z ein Sauerstoffatom ist.

4. Verbindung nach den Ansprüchen 1 bis 3, **dadurch gekennzeichnet, dass** sie durch die allgemeine Formel: 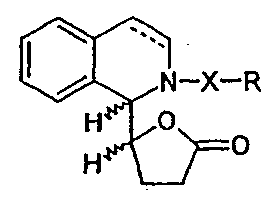 dargestellt wird, in der:
die Gruppe X eine Carbonyl-, Sulfonyl- oder CO₂-Gruppe darstellt und
die Gruppe R eine Alkyl-, Aryl-, Alkenyl- oder Aralkyl-Gruppe darstellt.

5. Verbindung nach den Ansprüchen 1 bis 4, **dadurch gekennzeichnet, dass** sie durch die Formel: 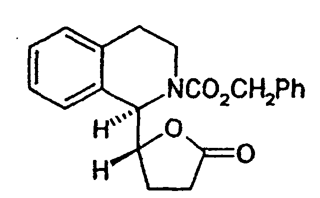 dargestellt wird.

6. Verbindung nach den Ansprüchen 1 bis 4, **dadurch gekennzeichnet, dass** sie durch die Formel: 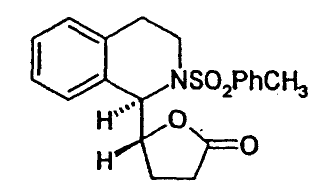 dargestellt wird.

7. Verfahren zur Herstellung von Verbindungen gemäß den Ansprüchen 1 bis 6, **dadurch gekennzeichnet, dass** die Verbindung der allgemeinen Formel: 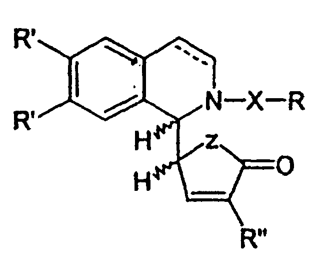 in der:
Z ein Schwefel-, Sauerstoffatom, NH, N-Alkyl oder Nboc darstellt,
die Gruppen R', die identisch oder voneinander verschieden sein können, jeweils ein Wasserstoffatom, eine OCH₃-Gruppe oder zusammen eine OCH₂O-Gruppe darstellen,
die Gruppe R" einen Wasserstoff oder eine CH₃-Gruppe darstellt,
die Gruppe X eine Carbonyl-, Sulfonyl- oder CO₂-Gruppe darstellt und
die Gruppe R eine Alkyl-, Aryl-, Alkenyl- oder Aralkyl-Gruppe darstellt,
selektiv in Anwesenheit von Pd-C durch H₂ hydriert wird, um die Verbindung nach Anspruch 1 zu ergeben.

8. Verfahren zur Herstellung nach Anspruch 7, **dadurch gekennzeichnet, dass** es einen vorgelagerten Schritt der Kondensation eines Silylenolethers, der durch die allgemeine Formel: 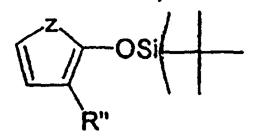 dargestellt wird, in der:
Z ein Schwefel-, Sauerstoffatom, NH, N-Alkyl oder Nboc darstellt und
R" ein Wasserstoffatom oder ein CH₃ darstellt,
mit einem Salz von 3,4-Dihydroisochinolin, das durch die folgende allgemeine Formel dargestellt wird: 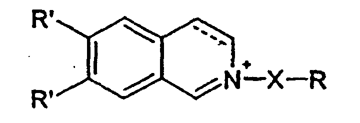
in der:
die Gruppen R', die identisch oder voneinander verschieden sein können, jeweils ein Wasserstoffatom, eine OCH₃-Gruppe oder zusammen eine OCH₂O-Gruppe darstellen,
die Gruppe X eine Carbonyl-, Sulfonyl- oder CO₂-Gruppe darstellt und
die Gruppe R eine Alkyl-, Aryl-, Alkenyl- oder Aralkyl-Gruppe darstellt,
in Anwesenheit eines Acylierungs- oder Sulfonylierungsmittels umfasst.

9. Pharmazeutische Zusammensetzung, die eine Verbindung nach den Ansprüchen 1 bis 6 und einen geeigneten pharmazeutischen Träger umfasst.

10. Verbindung gemäß den Ansprüchen 1 bis 6 und 9 für ihre Verwendung als Stimulans der Aktivität von γ-Aminobuttersäure, das über den GABA-A-Rezeptor des zentralen Nervensystems aktiv ist.

11. Verbindung nach den Ansprüchen 1 bis 6 und 9 für ihre Verwendung als Medikament.

12. Verwendung der Verbindungen nach den Ansprüchen 1 bis 6 und 9 für die Herstellung eines Medikaments, das für die Behandlung von Nervenstörungen bestimmt ist.

13. Verwendung nach Anspruch 12, **dadurch gekennzeichnet, dass** die Nervenstörungen vom Typ Epilepsie, Angst, Depression, Schlaf- und Gedächtnisstörungen, Panikattacken, Muskelkontraktionen, Schmerz, Alkohol- oder Benzodiazepin-Abhängigkeit oder psychotische Verhaltensweisen sind.

## Claims

1. Compound represented by the following general formula: 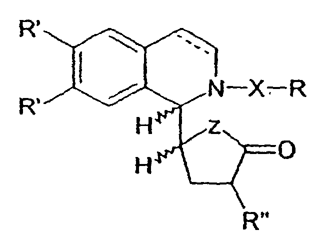 in which:
Z represents a sulphur atom, an oxygen atom, NH, N-alkyl or Nboc,
the groups R', which may be identical or different from one another, each represent a hydrogen atom or an OCH₃ group, or together represent an OCH₂O group,
the group R" represents a hydrogen or a CH₃ group,
the group X represents a carbonyl, sulphonyl or CO₂ group,
and the group R represents an alkyl, aryl, alkenyl or aralkyl group
in the form of their racemic mixture or of their optically pure isomers.

2. Compound according to Claim 1, **characterized in that** Z represents an oxygen atom or Nboc.

3. Compound according to Claims 1 and 2, **characterized in that** Z is an oxygen atom.

4. Compound according to Claims 1 to 3, **characterized in that** it is represented by the general formula: 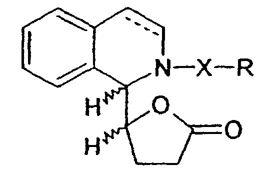 in which:
the group X represents a carbonyl, sulphonyl or CO₂ group
and the group R represents an alkyl, aryl, alkenyl or aralkyl group.

5. Compound according to Claims 1 to 4, **characterized in that** it is represented by the formula:

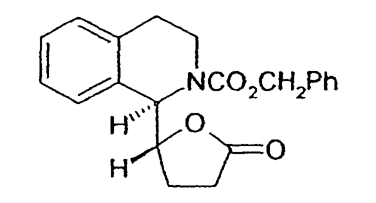

6. Compound according to Claims 1 to 4, **characterized in that** it is represented by the formula:

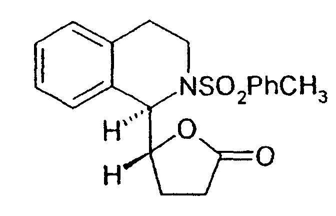

7. Method of preparing the compounds according to Claims 1 to 6, **characterized in that** the compound of general formula: 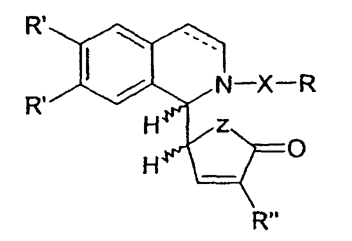 in which:
Z represents a sulphur atom, an oxygen atom, NH, N-alkyl or Nboc,
the groups R', which may be identical or different from one another, each represent a hydrogen atom or an OCH₃ group, or together represent an OCH₂O group,
the group R" represents a hydrogen or a CH₃ group,
the group X represents a carbonyl, sulphonyl or CO₂ group
and the group R represents an alkyl, aryl, alkenyl or aralkyl group
is selectively hydrogenated with H₂ in the presence of Pd-C to give the compound according to Claim 1.

8. Method of preparation according to Claim 7, **characterized in that** it comprises a preliminary step of condensing, in the presence of an acylating or sulphonylating agent of a silyl enol ether represented by the general formula: 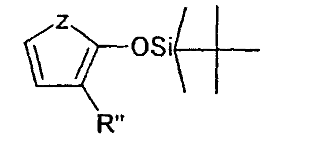 in which:
Z represents a sulphur atom, an oxygen atom, NH, N-alkyl or Nboc
and R" represents a hydrogen atom or a CH₃
with a salt of 3,4-dihydroisoquinoline represented by the following general formula: 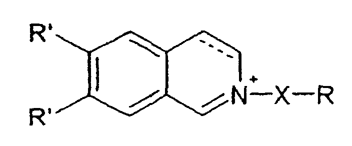
in which:
the groups R', which may be identical or different from one another, each represent a hydrogen atom or an OCH₃ group, or together represent an OCH₂O group,
the group X represents a carbonyl, sulphonyl or CO₂ group
and the group R represents an alkyl, aryl, alkenyl or aralkyl group.

9. Pharmaceutical composition comprising a compound according to Claims 1 to 6 and an appropriate pharmaceutical carrier.

10. Compound according to Claims 1 to 6 and 9, for its use as a stimulant of the activity of γ-aminobutyric acid acting via the GABA-A receptor of the central nervous system.

11. Compound according to Claims 1 to 6 and 9, for its use as a medicament.

12. Use of the compounds according to Claims 1 to 6 and 9, for the manufacture of a medicament for the treatment of nervous disorders.

13. Use according to Claim 12, **characterized in that** the nervous disorders are of the type including epilepsy, anxiety, depression, sleep and memory disorders, panic attacks, muscle contractions, pain, dependence on alcohol or on benzodiazepines or psychotic behaviour.
